# EUROPEAN PATENT APPLICATION

(11) **EP 4 748 497 A1**
(43) Date of publication of application: **27.05.2026**
(21) Application number: 25217516.1
(22) Date of filing: 20.11.2025
(51) Int. Cl.: B01L 3/00, B01L 1/00

(54) **MANIPULATION DEVICE FOR BIOLOGICAL PARTICLE AND MANUFACTURING METHOD THEREOF**

(30) Priority: 22.11.2024 TW 113145230
(71) Applicant: NEAT Biotech, Inc., Hsinchu City 300 (TW)
(72) Inventor: SHIAU, Jeng Huei, 302 Zhubei City (TW)
(74) Representative: Klöckner, Christoph

(57) **Abstract**

A manipulation device for a biological particle includes a substrate, a metal conductive layer, a working electrode, and a dielectric layer. The metal conductive layer is over the substrate. The working electrode is over and electrically in contact with the metal conductive layer. The dielectric layer covers the metal conductive layer. The dielectric layer has a first recess exposing the working electrode. A top surface of the working electrode is higher than a bottom of the first recess, and the dielectric layer is in contact with a sidewall of the working electrode.

## Description

### BACKGROUND

### Field of Invention

The present disclosure relates to a manipulation device for a biological particle and a manufacturing method thereof.

### Description of Related Art

In recent years, with the rapid development of biotechnology, manipulation techniques for various biological particles have emerged, and the biological particles that can be manipulated range from cell, virus, protein, to deoxyribonucleic acid (DNA), etc. Since the manipulation techniques for biological particles can locate individual biological particles, they can be used to measure and detect various physical, chemical, and biological properties. They can even manipulate specific target biological particles to achieve the goal of separating and purifying the specific biological particles. In quarantine applications, the rapid and accurate manipulation of biological particles allows accurate determination of the number of specific biological particles, such as the number of specific viruses, at an earlier stage, to improve quarantine accuracy.

Dielectrophoresis (DEP) technique is one of the manipulation techniques for biological particles. It can be widely applied to biological particles of varying sizes and demonstrates considerable manipulation effectiveness. In DEP manipulation technique, for example, an alternating current (AC) voltage is applied between two non-parallel electrodes so as to form a non-uniform electric field, thus generating a dielectricphoretic force that rapidly moves the target biological particle to a predetermined electrode position. Depending on the operating mode, DEP includes electrode-based dielectrophoresis (eDEP) and insulator-based dielectrophoresis (iDEP). Currently, eDEP is widely used in microfluidic devices due to its ability to generate high field gradients at a low applied voltage. High gradient fields are capable of manipulating larger particles because the DEP force is proportional to the square of the electric field strength and the volume of the target particle. However, higher gradients generated by the eDEP easily cause joule heating and decompose the electrodes, leading to the production of large amounts of toxic substances that damage cells and organelles.

For the foregoing reason, there is a need to provide a manipulation device for a biological particle to resolve the above-mentioned problem.

### SUMMARY

A manipulation device for a biological particle is provided. The manipulation device for the biological particle includes a substrate, a metal conductive layer, a working electrode, and at least one dielectric layer. The metal conductive layer is over the substrate. The working electrode is over the metal conductive layer and electrically in contact with the metal conductive layer. The at least one dielectric layer covers the metal conductive layer. The at least one dielectric layer has a first recess, the first recess exposes the working electrode, and a top surface of the working electrode is higher than a bottom of the first recess. The at least one dielectric layer is in contact with a sidewall of the working electrode.

In the foregoing, the at least one dielectric layer further has a second recess. The second recess extends downwards from the bottom of the first recess and surrounds the working electrode.

In the foregoing, a width of a top of the second recess is smaller than a width of the bottom of the first recess.

In the foregoing, a sidewall of the second recess is separated from a sidewall of the first recess by a bottom surface of the first recess.

In the foregoing, an upper portion of the sidewall of the working electrode protrudes from the at least one dielectric layer.

In the foregoing, the at least one dielectric layer includes a nitride layer and an oxide layer. The oxide layer is over the nitride layer.

In the foregoing, a top surface of the nitride layer of the at least one dielectric layer is higher than the top surface of the working electrode.

In the foregoing, the manipulation device further includes a via member. The via member is between the metal conductive layer and the working electrode.

In the foregoing, a width of the first recess is between 0.1 micrometers (µm) and 2 µm.

In the foregoing, a sidewall of the first recess surrounds the working electrode.

A manufacturing method of a manipulation device for a biological particle is provided. The manufacturing method includes forming a metal conductive layer over a substrate; forming a working electrode over the metal conductive layer and being electrically in contact with the metal conductive layer; and forming at least one dielectric layer covering the metal conductive layer. The at least one dielectric layer has a first recess exposing the working electrode, and a top surface of the working electrode is higher than a bottom of the first recess, wherein the at least one dielectric layer is in contact with a sidewall of the working electrode.

In the foregoing, the at least one dielectric layer further has a second recess, the second recess extends downwards from the bottom of the first recess and surrounds the working electrode.

In the foregoing, forming the at least one dielectric layer includes forming a nitride layer covering the metal conductive layer; and forming an oxide layer over the nitride layer.

In the foregoing, a top surface of the nitride layer of the at least one dielectric layer is higher than the top surface of the working electrode.

In the foregoing, the manufacturing method further includes forming an insulating layer over the metal conductive layer; and forming a via member in the insulating layer before forming the working electrode.

It is to be understood that both the foregoing general description and the following detailed description are by examples, and are intended to provide further explanation of the disclosure as claimed.

### BRIEF DESCRIPTION OF THE DRAWINGS

The accompanying drawings are included to provide a further understanding of the present disclosure, and are incorporated in and constitute a part of this specification. The drawings illustrate embodiments of the present disclosure and, together with the description, serve to explain the principles of the present disclosure. In the drawings,
Fig. 1 depicts a cross-sectional view of a manipulation device according to some embodiments of the present disclosure;
Fig. 2 to Fig. 11 depict cross-sectional views of a process of forming a manipulation device according to some embodiments of the present disclosure;
Fig. 12 depicts a top view of a manipulation device according to some embodiments of the present disclosure; and
Fig. 13 depicts a top view of a manipulation device according to some other embodiments of the present disclosure.

### DESCRIPTION OF THE EMBODIMENTS

Reference will now be made in detail to the present embodiments of the disclosure, examples of which are illustrated in the accompanying drawings. However, the embodiments provided herein are intended as illustrative only since numerous modifications and variations therein will be apparent to those skilled in the art. Wherever possible, the same reference numbers are used in the drawings and the description to refer to the same or like parts for better understanding.

Some embodiments of the present disclosure related to a manipulation device for a biological particle. The manipulation device according to the present disclosure can be applied to bioparticle manipulation technology and the field of electrode-based dielectrophoresis (eDEP). The biological particle may include a cell, a bacterium, a virus, a protein, a deoxyribonucleic acid (DNA), etc. When manipulating the biological particle, the manipulation device for the biological particle according to the present disclosure can avoid direct contacts between the biological particle and electrodes during the eDEP is performed, thus significantly reducing the damages caused by joule heating or electrochemically decomposed substances to the biological particle, such as the cell, so as to enhance viability of the biological particle.

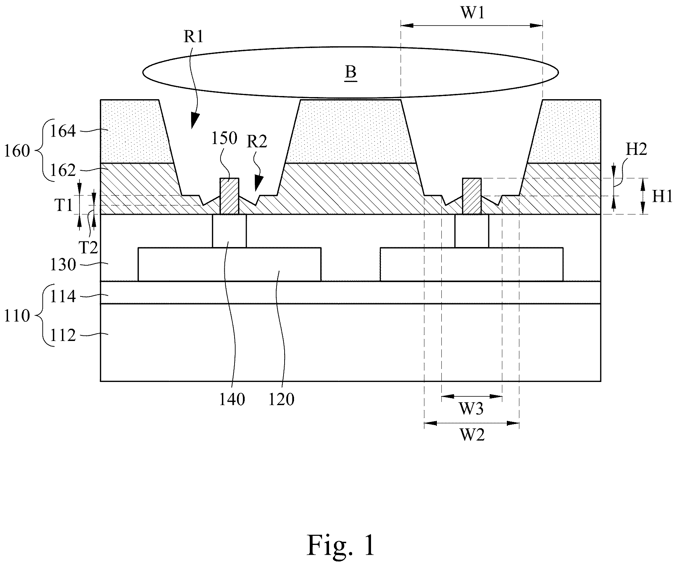

Fig. 1 depicts a cross-sectional view of a manipulation device according to some embodiments of the present disclosure. A description is provided with reference to Fig. 1. The manipulation device includes a substrate 110, a metal conductive layer 120, an insulating layer 130, via members 140, working electrodes 150, and a dielectric layer 160. The manipulation device can be configured to adsorb a target biological particle B. In some embodiments, the target biological particle B may be a biological particle, which may include a cell, a bacterium, a virus, a protein, a deoxyribonucleic acid (DNA), etc.

In some embodiments, the substrate 110 includes a base material 112 and an insulating layer 114. The substrate 110 may include, but is not limited to, a semiconductor material, such as gallium nitride (GaN), silicon carbide (SiC), silicon germanium (SiGe), germanium, or a combination thereof. The base material 112 may be, for example, a silicon base material. Depending on the design requirements of the technical field, the base material 112 may include various doping configurations. In some embodiments, the base material 112 may be a heavily doped, low-resistivity semiconductor base material. In some other embodiments, the substrate 110 is a glass substrate and does not have the insulating layer 114.

The insulating layer 114 is over the base material 112. In some embodiments, the insulating layer 114 may include, but is not limited to, an oxide, a nitride, an oxynitride, or a combination thereof, such as silicon oxide, silicon nitride, silicon oxynitride. The insulating layer 114 is made of a low-k material, so that the manipulation device has a good insulation property. In some embodiments, a thickness of the insulating layer 114 is from about 0.02 micrometers (µm) to about 0.6 µm, such as about 0.10 µm, about 0.15 µm, or about 0.20 µm.

The metal conductive layer 120 is over the substrate 110. In some embodiments, the metal conductive layer 120 may include, but is not limited to, titanium (Ti), nickel (Ni), silver (Ag), aluminum (Al), aluminum-copper (AlCu) alloy, aluminum-silicon-copper (AlSiCu) alloy, or a combination thereof. In some embodiments, a thickness of the metal conductive layer 120 is from about 0.02 µm to about 0.7 µm, such as about 0.1 µm, about 0.2 µm, about 0.3 µm, about 0.4 µm, about 0.5 µm, or about 0.6 µm.

The insulating layer 130 covers the metal conductive layer 120 and is in contact with a top surface and sidewalls of the metal conductive layer 120. In some embodiments, the insulating layer 130 may include, but is not limited to, an oxide, a nitride, an oxynitride, or a combination thereof, or a compound thereof, such as silicon oxide, silicon nitride, silicon oxynitride. In some embodiments, a material of the insulating layer 114 is the same as a material of the insulating layer 130. In some embodiments, the material of the insulating layer 114 is different form the material of the insulating layer 130.

The via members 140 are in the insulating layer 130 and over the metal conductive layer 120. A width of each of the via members 140 may be smaller than a width of the metal conductive layer 120, and the insulating layer 130 surrounds the via members 140. In some embodiments, the via member 140 may include, but is not limited to, tungsten (W), copper (Cu), or a combination thereof. In some embodiments, a thickness of each of the via members 140 is from about 0.2 µm to about 0.6 µm.

The working electrodes 150 are over the metal conductive layer 120 and electrically in contact with the via members 140 and the metal conductive layer 120. That is, the via members 140 are between the metal conductive layer 120 and the working electrodes 150. A width of the working electrode 150 may be smaller than the width of the via member 140. Each of the working electrodes 150 having a height H1 protrudes from the via member 140 correspondingly. In some embodiments, the height H1 of the working electrode 150 is from about 0.05 µm to about 0.5 µm, such as about 0.05 µm, about 0.1 µm, about 0.2 µm, about 0.3 µm, or about 0.4 µm. In some embodiments, the width of the working electrode 150 is from about 0.08 µm to about 0.4 µm, such as about 0.08 µm, about 0.1 µm, about 0.2 µm, or about 0.3 µm. In some embodiments, the working electrode 150 has an aspect ratio, which is from about 0.125 to about 7.5, such as about 0.2 or about 0.3.

In some embodiments of the present disclosure, a shape of the working electrode 150 may be a cylinder or a regular polygonal prism, such as a regular triangular prism, a regular square prism, a regular pentagonal prism, a regular hexagonal prism, or a regular octagonal prism. In some embodiments, the working electrode 150 may include, but is not limited to, tantalum (Ta), tantalum nitride (TaN), copper (Cu), titanium (Ti), titanium nitride (TiN), tungsten (W), titanium (Ti), nickel (Ni), silver (Ag), aluminum (Al), aluminum-copper (AlCu) alloy, aluminum-silicon-copper (AlSiCu) alloy, or a combination thereof. In some embodiments, a material of the working electrode 150 is preferably titanium nitride (TiN).

The dielectric layer 160 covers the insulating layer 130, the via members 140, and the working electrodes 150. The dielectric layer 160 has recesses R1. The recesses R1 expose the working electrodes 150, and top surfaces of the working electrodes 150 are higher than bottoms of the recesses R1. A thickness T1 greater than zero exists between the bottoms of the recesses R1 and a bottom of the dielectric layer 160. In other words, there is no dielectric layer 160 directly over the working electrodes 150. In some embodiments, the thickness T1 between the bottoms of the recesses R1 and the bottom of the dielectric layer 160 is from about 0.03 µm to about 0.15 µm. In some embodiments, each of the recesses R1 may have a substantially circular top view profile. In some embodiments, tops of the recesses R1 have a width W1 (or a diameter), and the bottoms of recesses R1 have a width W2. The width W1 is greater than the width W2. In other words, in the cross-sectional view of Fig. 1, each of the recesses R1 has a profile that tapers downwards. In some embodiments, the width W1 is a maximum width of the recesses R1.

In some embodiments, the maximum width (such as the width W1) of the recesses R1 is smaller than a size of the target biological particle B. In some embodiments, the width W1 of the recesses R1 is between about 0.1 µm and 2 µm. In some embodiments, the top surfaces of the working electrodes 150 are higher than the bottoms of the recesses R1 by a height H2, and the height H2 is from about 0.05 µm to about 0.2 µm. That is, the dielectric layer 160 is in contact with sidewalls of the working electrodes 150, and upper portions of the sidewalls of the working electrodes 150 protrude from the dielectric layer 160.

The dielectric layer 160 further has recesses R2. The recesses R2 are on the bottoms of the recesses R1 and surround the working electrodes 150, and bottoms of the recesses R2 are lower than the bottoms of the recesses R1. In greater detail, the recesses R2 extend downwards from bottom surfaces of the recesses R1, so that sidewalls of the recesses R2 are separated from sidewalls of the recesses R1 by the bottom surfaces of the recesses R1. In some embodiments, tops of the recesses R2 have a width W3. The width W3 is smaller than the width W2 of the bottoms of the recesses R1. A thickness T2 greater than zero also exists between the bottoms of the recesses R2 and the bottom of the dielectric layer 160. In some embodiments, the thickness T2 between the bottoms of the recesses R2 and the bottom of the dielectric layer 160 is from about 0.02 µm to about 0.05 µm.

The dielectric layer 160 may be made of a dielectric material. In some embodiments, the dielectric layer 160 may include, but is not limited to, an oxide, a nitride, an oxynitride, or a combination thereof, or a compound thereof, such as silicon oxide, silicon nitride, silicon oxynitride. For example, the dielectric layer 160 may include a nitride layer 162 and an oxide layer 164. The oxide layer 164 is over the nitride layer 162.

In the manipulation technique of the biological particle, a solution containing the target biological particle B with specific conductivity is placed on a manipulation device which is made of non-parallel electrode arrangement designed based on DEP theory, and a setting AC voltage is applied to the working electrodes 150 of the device to attract the target biological particle B to vicinities of the working electrodes 150. Then, processes, such as detection, separation, electroporation, etc., can be performed on the target biological particle B. The recess R1 according to some embodiments of the present disclosure can be used to significantly enhance the viability of the target biological particle B when using the eDEP. In greater detail, the recesses R1 of the dielectric layer 160 expose the working electrodes 150. Therefore, under the circumstance that the eDEP is used to perform the bioparticle manipulation technique, when the target biological particle B is pulled by the dielectricphoretic force to move towards the working electrodes 150 where the electric field is concentrated or divergent, there is ultimately no substantial contacts between the target biological particle B and the working electrodes 150, thus significantly reducing damages caused by the operating electrical signal and joule heating on the working electrodes 150 to the target biological particle B. In addition, since the width W1 of the recesses R1 of the dielectric layer 160 is smaller than the size of the target biological particle B, it is ensured that the target biological particle B does not fall into the recesses R1 and is not in contact with the working electrodes 150.

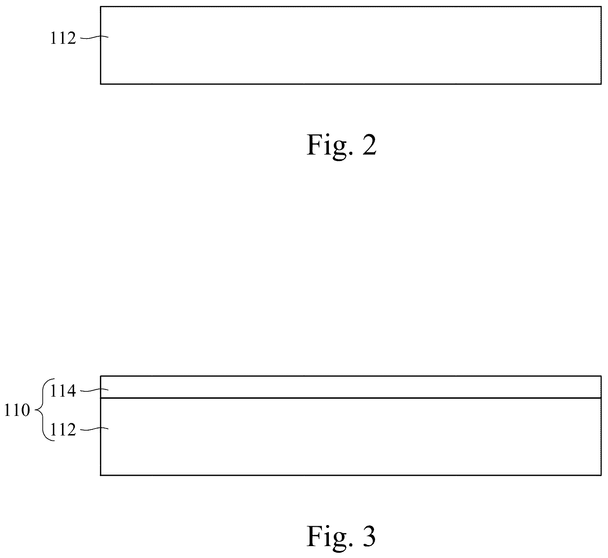

Fig. 2 to Fig. 11 depict cross-sectional views of a process of forming a manipulation device according to some embodiments of the present disclosure. A description is provided with reference to Fig. 2 to Fig. 3. A substrate 110 is provided. In some embodiments, the substrate 110 includes the base material 112 and the insulating layer 114. The insulating layer 114 is formed over the base material 112. The insulating layer 114 can be formed by atomic layer deposition (ALD), physical vapor deposition (PVD), chemical vapor deposition (CVD), chemical oxidation, thermal oxidation, and/or other suitable method. In some embodiments, the insulating layer 114 may include, but is not limited to, an oxide, a nitride, an oxynitride, or a combination thereof, such as silicon oxide, silicon nitride, silicon oxynitride.

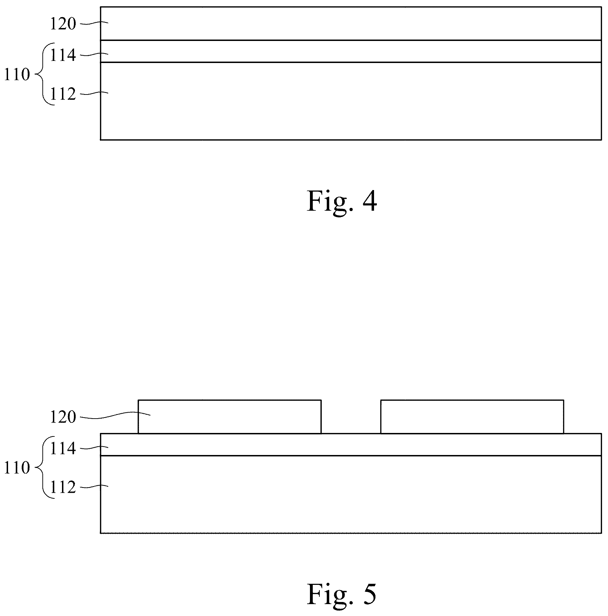

A description is provided with reference to Fig. 4. The metal conductive layer 120 is formed over the insulating layer 114 of the substrate 110. In some embodiments, the metal conductive layer 120 can be formed by using PVD, CVD, electron beam evaporation, sputtering, electroplating, and/or other suitable process. In some embodiments, the metal conductive layer 120 may include, but is not limited to, titanium (Ti), nickel (Ni), silver (Ag), aluminum (Al), aluminum-copper (AlCu) alloy, aluminum-silicon-copper (AlSiCu) alloy, or a combination thereof.

A description is provided with reference to Fig. 5. A patterning process is performed on the metal conductive layer 120. In greater detail, a patterned photoresist layer (not shown in the figure) can be formed over the metal conductive layer 120 by using a mask and a photolithography process. This photoresist layer may be, for example, a positive photoresist or a negative photoresist. Next, an etching process is performed on the metal conductive layer 120 by using the patterned photoresist layer to expose part of a top surface of the underlying insulating layer 114.

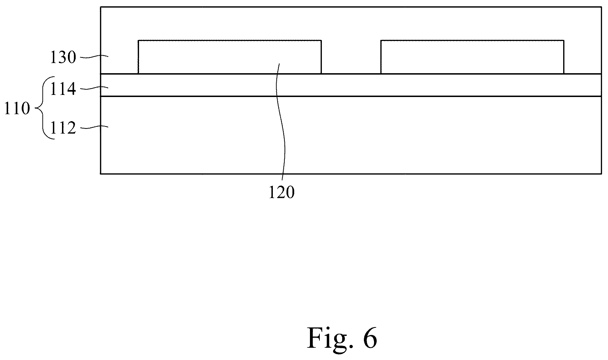

A description is provided with reference to Fig. 6. The insulating layer 130 is deposited over the insulating layer 114 and the metal conductive layer 120. In some embodiments, the insulating layer 130 may have multiple layers, and materials of the multiple layers are different from each other. In some embodiments, the insulating layer 130 may have the multiple layers, and the materials of the multiple layers are the same as each other. In some embodiments, the insulating layer 130 may include, but is not limited to, an oxide, a nitride, an oxynitride, or a combination thereof, such as silicon oxide, silicon nitride, silicon oxynitride. In one embodiments, the insulating layer 130 is tetraethoxysilane. In some embodiments, the insulating layer 130 can be formed by using PVD, CVD, plasma enhanced chemical vapor deposition (PECVD) and/or other suitable process.

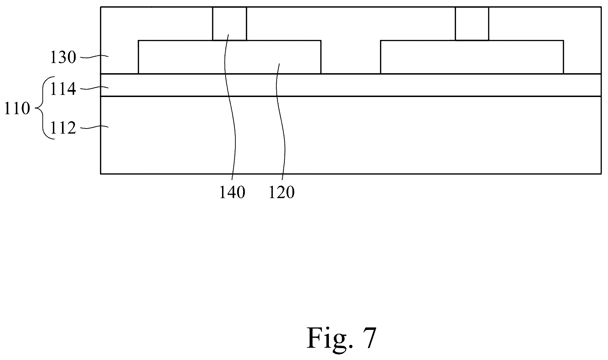

A description is provided with reference to Fig. 7. Via members 140 are formed in the insulating layer 130. In greater detail, vias can be formed in the insulating layer 130, and a conductive material is filled in the vias. Finally, a planarization process is performed to remove excess material overflowing the vias to form the via members 140 in the insulating layer 130. In some embodiments, each of the via members 140 may include, but is not limited to, tungsten (W), copper (Cu), or a combination thereof.

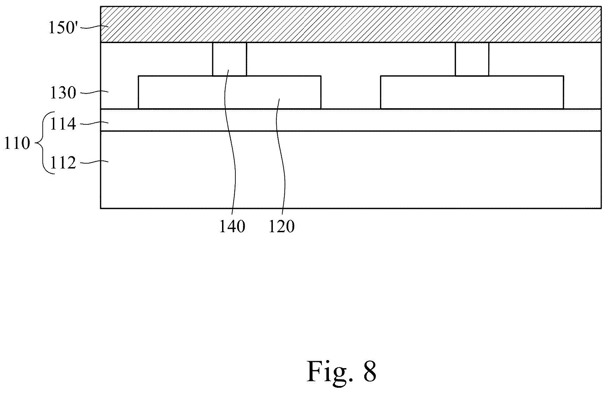

A description is provided with reference to Fig. 8. A conductive layer 150' is deposited over the insulating layer 130 and the via members 140. In some embodiments, the conductive layer 150' can be formed by using PVD, CVD, electron beam evaporation, sputtering, electroplating, and/or other suitable process. In some embodiments, the conductive layer 150' may include, but is not limited to, tantalum (Ta), tantalum nitride (TaN), copper (Cu), titanium (Ti), titanium nitride (TiN), tungsten (W), or a combination thereof.

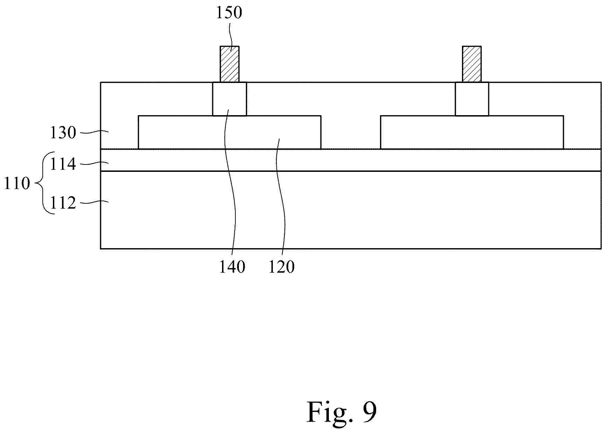

Then, a description is provided with reference to Fig. 9. A patterning process is performed on the conductive layer 150' to form the plurality of working electrodes 150. In greater detail, a patterned photoresist layer (not shown in the figure) can be formed over the conductive layer 150' by using a mask and a photolithography process. This photoresist layer may be, for example, a positive photoresist or a negative photoresist. Next, an etching process is performed on the conductive layer 150' by using the patterned photoresist layer to form the plurality of working electrodes 150 and expose a top surface of the insulating layer 130 and part of top surfaces of the via members 140. Each of the working electrodes 150 has a height H1.

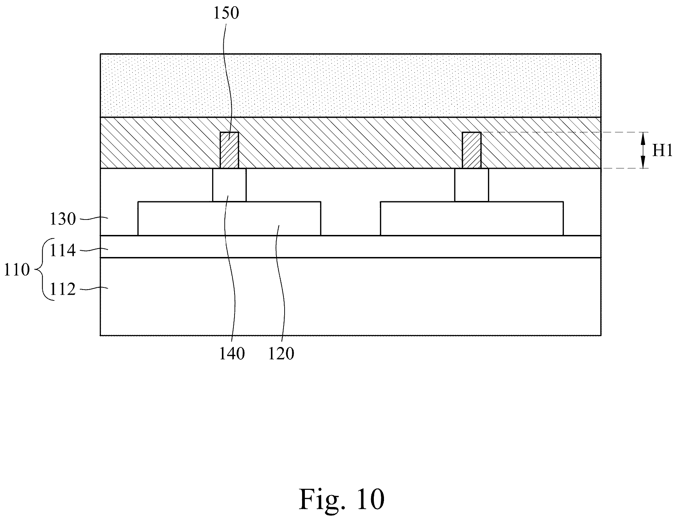

A description is provided with reference to Fig. 10. The dielectric layer 160 is formed over the insulating layer 130 and the working electrodes 150. In some embodiments, the insulating layer 130 may have the multiple layers, and the materials of the multiple layers are different from each other. In some embodiments, the dielectric layer 160 may have the multiple layers, and the materials of the multiple layers are the same as each other. In some embodiments, the insulating layer 130 may include, but is not limited to, the oxide, the nitride, the oxynitride, or the combination thereof, such as silicon oxide, silicon nitride, silicon oxynitride. In one embodiment, the insulating layer 130 is tetraethoxysilane. For example, the dielectric layer 160 may include the nitride layer 162 and the oxide layer 164, and the oxide layer 164 is over the nitride layer 162. The nitride layer 162 completely covers the working electrodes 150. The nitride layer 162 is used to prevent the working electrodes 150 from being oxidized simultaneously when the oxide layer 164 is formed, thereby affecting a resistance value of the working electrodes 150. In some embodiments, the dielectric layer 160 can be formed by using PVD, CVD, plasma enhanced CVD (PECVD) and/or other suitable process. In some embodiments, a whole top surface of the nitride layer 162 is higher than top surfaces of the working electrodes 150, as shown in Fig. 10. In some other embodiments, only part of the top surface of the nitride layer 162 is higher than the top surfaces of the working electrodes 150. For example, the nitride layer 162 is a thin layer conformal to the insulating layer 130 and the working electrodes 150, so that only the top surface of the nitride layer 162 near tops of the working electrodes 150 is higher than the top surfaces of the working electrodes 150.

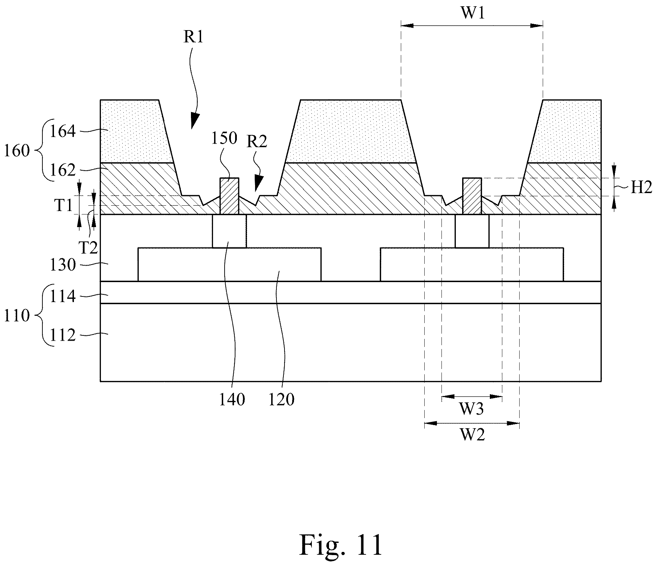

A description is provided with reference to Fig. 11. The recesses R1 are formed in the dielectric layer 160. In greater detail, a patterned photoresist layer (not shown in the figure) can be formed over the dielectric layer 160 by using a mask and a photolithography process. This photoresist layer may be, for example, a positive photoresist or a negative photoresist. Next, an etching process is performed on the dielectric layer 160 by using the patterned photoresist layer to expose the working electrodes 150. Since the etching process has etching selectivity for the dielectric layer 160 and the working electrodes 150, the working electrodes 150 are only slightly etched when forming the recesses R1 (that is, by using the etching process, the rate of etching the dielectric layer 160 is much higher than the rate of etching the working electrodes 150). In some embodiments, after the recesses R1 are formed, the top surface of the nitride layer 162 of the dielectric layer 160 may be higher than the top surfaces of the working electrodes 150. In some other embodiments, after the recesses R1 are formed, the top surface of the nitride layer 162 of the dielectric layer 160 may be lower than the top surfaces of the working electrodes 150. Tops of the recesses R1 have the width W1 (or the diameter), and bottoms of recesses R1 have the width W2. The width W1 is greater than the width W2. The width W1 of the recesses R1 is smaller than a size of a target biological particle, so that the target biological particle is not in direct contact with the working electrodes 150. When performing the etching process, the dielectric layer 160 is not completely etched through, and thus the thickness T1 exists between the bottoms of the recesses R1 and a bottom of the dielectric layer 160. That is, upper portions of the working electrodes 150 are exposed to the environment, whereas lower portions of the working electrodes 150 are covered by the dielectric layer 160, and the top surfaces of the working electrodes 150 are higher than the bottoms of the recesses R1 by the height H2. In some embodiments, when the dielectric layer 160 is etched to form the recesses R1, the recesses R1 may not be completely flat due to the existence of the working electrodes 150. For example, the etching process will etch the material around the working electrodes 150 at a faster rate, so that the recesses R2 surrounding the working electrodes 150 will be formed on the bottoms of the recesses R1. The thickness T2 greater than zero also exists between bottoms of the recesses R2 and the bottom of the dielectric layer 160. Tops of the recesses R2 have the width W3. The width W3 is smaller than the width W2 of the bottoms of the recesses R1.

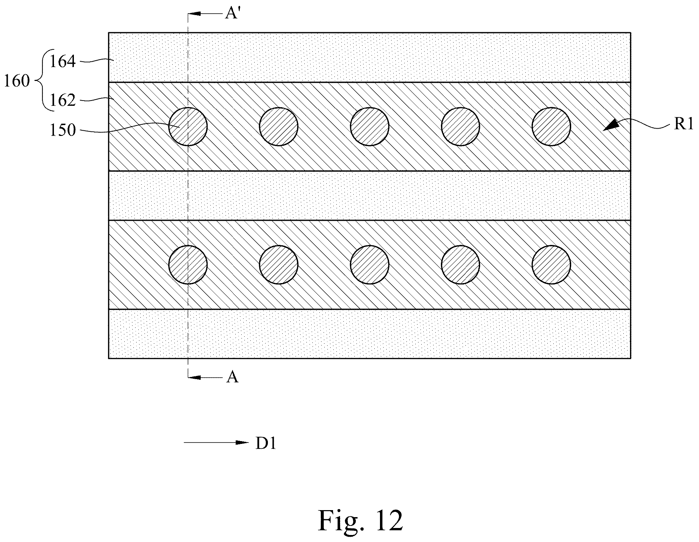

Fig. 12 depicts a top view of a manipulation device according to some embodiments of the present disclosure. Fig. 1 to Fig. 11 may be cross-sectional views taken along line A-A' of Fig. 12. In some embodiments, one recess R1 may correspond to a plurality of working electrodes 150, and a lengthwise direction of the recesses R1 may be along a first direction D1, and the working electrodes 150 in each of the recesses R1 are also arranged along the first direction D1. That is, a sidewall of each of the recesses R1 is on two sides of the working electrodes 150.

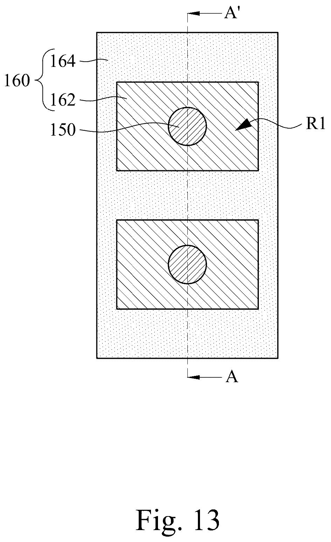

Fig. 13 depicts a top view of a manipulation device according to some other embodiments of the present disclosure. Fig. 1 to Fig. 11 may be cross-sectional views taken along line A-A' of Fig. 13. In some embodiments, one recess R1 may correspond to one working electrode 150. That is, a sidewall of each of the recesses R1 surrounds the working electrode 150.

It should be noted that the present disclosure does not limit the corresponding relationships and arrangement methods of the recesses R1 and the working electrodes 150. As long as the width W1 of the recesses R1 is smaller than the size of the target biological particle so that the target biological particle does not fall into the recesses R1 to be in direct contact with the working electrodes 150, then the recesses R1 can be used to improve the dielectricphoretic force between the target biological particle and the working electrodes 150 without damaging the target biological particle. The present disclosure also does not limit a shape of the recess R1. In some embodiments, the shape of the recess R1 may be a square, a circle, triangle, etc.

In summary, the manipulation device according to some embodiments of the present disclosure includes the dielectric layer having the recesses. The recesses expose the working electrodes, so there is ultimately no substantial contacts between the target biological particle and the working electrodes when the target biological particle is pulled by the dielectricphoretic force to move towards the working electrodes where the electric field is concentrated or divergent, under the circumstance that the eDEP is used to perform the bioparticle manipulation technique. The damages caused by the operating electrical signal and joule heating on the working electrodes to the target biological particle B are significantly reduced. Additionally, since the size of the recesses is smaller than the size of the target biological particle, the recesses can ensure that the working electrodes are not in contact with and do not damage the target biological particle.

## Claims

1. A manipulation device for a biological particle comprising:
a substrate (110);
a metal conductive layer (120) over the substrate (110);
a working electrode (150) over the metal conductive layer (120) and being electrically in contact with the metal conductive layer (120); and
at least one dielectric layer (160) covering the metal conductive layer (120), the at least one dielectric layer (160) having a first recess (R1), the first recess (R1) exposing the working electrode (150), and a top surface of the working electrode (150) being higher than a bottom of the first recess (R1), wherein the at least one dielectric layer (160) is in contact with a sidewall of the working electrode (150).

2. The manipulation device of claim 1, wherein the at least one dielectric layer (160) further has a second recess (R2), the second recess (R2) extends downwards from the bottom of the first recess (R1) and surrounds the working electrode (150).

3. The manipulation device of claim 2, wherein a width of a top of the second recess (R2) is smaller than a width of the bottom of the first recess (R1).

4. The manipulation device of claim 2 or 3, wherein a sidewall of the second recess (R2) is separated from a sidewall of the first recess (R1) by a bottom surface of the first recess (R1).

5. The manipulation device of claim 1, 2, 3 or 4, wherein an upper portion of the sidewall of the working electrode (150) protrudes from the at least one dielectric layer (160).

6. The manipulation device of claim 1, 2, 3, 4 or 5, wherein the at least one dielectric layer (160) comprises:
a nitride layer (162); and
an oxide layer (164) over the nitride layer (162).

7. The manipulation device of claim 6, wherein a top surface of the nitride layer (162) of the at least one dielectric layer (160) is higher than the top surface of the working electrode (150).

8. The manipulation device of claim 1, 2, 3, 4, 5, 6, or 7 further comprises:
a via member (140) between the metal conductive layer (120) and the working electrode (150).

9. The manipulation device of claim 1, 2, 3, 4, 5, 6, 7 or 8, wherein a width of the first recess (R1) is between 0.1 micrometers (µm) and 2 µm.

10. The manipulation device of claim 1, 2, 3, 4, 5, 6, 7, 8 or 9, wherein a sidewall of the first recess (R1) surrounds the working electrode (150).

11. A manufacturing method of a manipulation device for a biological particle comprising:
forming a metal conductive layer (120) over a substrate (110);
forming a working electrode (150) over the metal conductive layer (120) and being electrically in contact with the metal conductive layer (120); and
forming at least one dielectric layer (160) covering the metal conductive layer (120), the at least one dielectric layer (160) having a first recess (R1), the first recess (R1) exposing the working electrode (150), and a top surface of the working electrode (150) being higher than a bottom of the first recess (R1), wherein the at least one dielectric layer (160) is in contact with a sidewall of the working electrode (150).

12. The manufacturing method of claim 11, wherein the at least one dielectric layer (160) further has a second recess (R2), the second recess (R2) extends downwards from the bottom of the first recess (R1) and surrounds the working electrode (150).

13. The manufacturing method of claim 11 or 12, wherein forming the at least one dielectric layer (160) comprises:
forming a nitride layer (162) covering the metal conductive layer (120); and
forming an oxide layer (164) over the nitride layer (162).

14. The manufacturing method of claim 13, wherein a top surface of the nitride layer (162) of the at least one dielectric layer (160) is higher than the top surface of the working electrode (150).

15. The manufacturing method of claim 13, further comprises:
forming an insulating layer (130) over the metal conductive layer (120); and
forming a via member (140) in the insulating layer before forming the working electrode (150).
